# EUROPEAN PATENT SPECIFICATION

(11) **EP 2 219 733 B1**
(45) Date of publication and mention of the grant of the patent: **07.01.2015**
(21) Application number: 08840163.3
(22) Date of filing: 16.10.2008
(51) Int. Cl.: A61N 5/10, A61F 2/10

(54) **METHOD OF TREATMENT OF DERMIS AND USES OF SAID DERMIS**
VERFAHREN ZUR BEHANDLUNG VON DERMIS UND DEREN VERWENDUNGEN
PROCÉDÉ DE TRAITEMENT DE DERME ET SON UTILISATION

(30) Priority: 19.10.2007 IT BO20070702
(43) Date of publication of application: 25.08.2010
(73) Proprietor: A.U.S.L. - Azienda Unità Sanitaria Locale Di Cesena, 47521 Cesena (IT); Istituto Ortopedico Rizzoli, 40136 Bologna (IT)
(72) Inventor: MELANDRI, Davide, I-48100 Ravenna (IT); BONDIOLI, Elena, I-47900 Rimini (IT); GIARDINO, Roberto, I-40136 Bologna (IT); FINI, Milena, I-40068 San Lazzaro Savena-Bologna (IT)
(74) Representative: Biggi, Cristina
(86) International application number: PCT/IB2008/002753
(87) International publication number: WO 2009/050571

(56) References cited:
- WO-A1-99/44533
- US-A1- 2003 158 607
- LIN ET AL: "Fabrication and evaluation of auto-stripped tri-layer wound dressing for extensive burn injury" MATERIALS CHEMISTRY AND PHYSICS, ELSEVIER, vol. 102, no. 2-3, 8 March 2007 (2007-03-08), pages 152-158, XP005917309 ISSN: 0254-0584
- BERTHOD F ET AL: "Optimization of thickness, pore size and mechanical properties of a biomaterial designed for deep burn coverage" CLINICAL MATERIALS, ELSEVIER, GB, vol. 15, no. 4, 1 January 1994 (1994-01-01), pages 259-265, XP023261239 ISSN: 0267-6605 [retrieved on 1994-01-01]

## Description

The present invention relates to a method of treatment of dermis, and relative uses of the obtained dermis More particularly, the present invention relates to a method of treatment of dermis for selectively preserving protein-based extracellular matrixes and eliminating and necrotizing cells, such as fibroblasts, macrophages, mast cells and specific cells of organs and tissues, responsible for immune and rejection reactions in homologous and heterologous transplantations.

The connective tissue transplantation is an operation which is often capable of saving the life of a subject.

A typical example is given by the skin transplantation, which is used in widespread third- and second-degree burns of the body. Subjects that experience burns in most of the body risk their life as the lack of the cutaneous coating causes the loss of liquids and proteins, dramatically lowers the body temperature and directly exposes the organism to physical and microbiological agents.

When burns are not very wide, it is possible to carry out skin autotransplants, by withdrawing the skin from the unburnt parts of the same subject and implanting it on the burns.

In autologous-type transplantations, the tissue required for the transplantation is directly withdrawn from the subject that will receive the transplanted tissue.

In these kinds of transplantation, complications due to immune and rejection reactions are not known, as the transplanted tissue is recognized by the organism and therefore is not attacked by the immune defenses. However, if the tissue withdrawn for the transplantation, for example the dermis, is used for the reconstruction of tissues of a different origins, for example tendons, within the same organism, it is possible that difficulties of transplanted tissue integration and post-transplantation recovery occur.

Furthermore, in some cases, autologous transplantations can not be practiced. In fact, for example in very wide burns, there is not enough undamaged skin for carrying out an autotransplant.

In these cases, homologous or heterologous transplantations are used, namely transplantations in which the donor is of the same species of the recipient but is not the recipient himself, for example he is a relative, a friend or a stranger, or in which the donor is of a different species from the one of the recipient. In case the recipient belongs to the human species, the donor can be a mammal, for example belonging to the swine, bovine or equine species.

These transplantations, however, are subjected to serious post-operative complications due to immunological and rejection reactions of the transplanted tissue. The rejection is that complex of biological reactions on the basis of which the organism tends to refuse the transplanted tissue, by recognizing it as a foreign tissue.

In order to obviate this serious drawback, methods were developed aimed to the obtainment of the immune tolerance, namely the biological acceptance from the organism receiving the foreign tissue which has been grafted therein.

A first kind of method foresees the administration of immunosuppressive drugs, for example cyclosporin, to the subject that has been subjected to the transplantation. Such methodology, if from one side decreases the rejection probability, from the other side exposes the transplanted subject, however already weakened by the experienced operation, to the possible contraction of infections which cannot be autonomously contrasted by the organism of the transplanted subject, as the whole immune system is depressed by the administered drugs.

Another type of method developed trying to avoid the rejection of the transplanted tissue foresees the treatment of the tissue withdrawn by the donor before grafting the same within the receiving subject.

Such methodology is directed from one side to stabilize the protein and collagenous structures of the extracellular structure of the withdrawn tissue and, from the other side, to mask or eliminate the antigen agents existing in the withdrawn tissue.

Several scientific works have proved the possibility of treating the tissue in the way above shown in order to render the same "acellular" and therefore avoid immune or rejection reactions.

A known example of such work is given by a method for obtaining a dermal acellular implant obtained by skin of a human corpse.

Such method includes the elimination of the cells responsible for the immune reactions from the tissue through the use of cleaning substances, such as polyoxyethylene, sodium deoxycholate and the like. Alternatively, instead of cleaning substances, the use of enzymes, or particular salts, is described. Successively, such method includes the step of placing the treated tissue in a cryoprotective liquid and dehydrate the same, in a predetermined percentage as a function of the weight of the tissue, during the cryof reezing.

The cryofreezing allows to preserve intact the extra-cellular matrix of the tissue.

The cryofrozen tissue is stored in a hydrating solution for preventing microbial contaminations of the tissue.

The tissue thus obtained is thawed and rehydrated before the transplantation and, once transplanted, is rapidly re-vascularized by the recipient's blood. Despite the tissue obtained by the method shortly described allows to prevent and limit post-operative complications due to the rejection, the method for obtaining such tissue is laborious and difficult to repeat as, for example, it requires lots of skill and sensitivity for correctly dehydrating, and in the right quantity, the partly pre-treated tissue.
WO 99/44533 A1 teaches enzymatic coating of soft tissue after irradiation rather than before.

In this context, the object of the present invention is to provide a method of treatment of dermis which allows to selectively preserve the extracellular matrix of the tissue and which eliminates or necrotizes the cells responsible for immune and rejection reactions in homologous and heterologous transplantations.

In particular, the object of the present invention is to provide a method of treatment of dermis which is easily implementable and repeatable.

According to the invention, such objects are attained by a method of treatment of a dermis including the steps exposed in one or more of the appended claims.

Features of the invention according to the above objects are clearly verifiable by the content of the claims given below, and the advantages of the same will result better apparent in the detailed description of a preferred embodiment of the invention.

The method of treatment of the present invention will be described with reference to human dermis withdrawn from the corpse of a donor. Alternatively, the dermis can be withdrawn from a living donor, for example a relative, a friend of the patient or from a stranger, or from a donor belonging to another species, for example from a mammal. The mammal is preferably selected among the swine, bovine, equine species.

The method described below can also be applied to dermis withdrawn from a subject (patient) and implanted to the same individual for the reconstruction of tissues of a different origin. In this case, it is a matter of an autotransplant and/or autodonation; for example, a portion of dermis can be withdrawn from a patient and, after a treatment with the method described below, used as a three-dimensional extracellular matrix for the reconstruction of a tendon, or other tissue of the body of the same patient.

The treatment before the implant is necessary for neutralizing the cells of the dermis which are incompatible with the cells of the tendon and could give rise to phenomena of a slow and impossible integration or recovery of the implanted tendon. An embodiment of the invention is described below in detail.

From the trunk of the donor a flap of dermis having a varying thickness and comprised between 0.3 and 3 mm, preferably from 0.3 to 1.5 mm, is removed, preferably with an electric dermatome.

The collected flap is placed within a sterile pot containing normal saline, in particular a 0.9% sodium chloride solution, for the transport to the treatment station.

Within a laminar flow sterile hood, which allows to ensure adequate sterility and safety conditions, the collected flap is carefully laid within a flask for cell cultures, adhering the upper part of the dermis to the surface of the flask. The dermis is therefore covered with an enzymatic solution capable of phagocytizing, at least partly, fibroblasts, macrophages, mast cells and other cells responsible for immune and rejection reactions in transplantations.

The dermis thus dipped is left in the enzymatic solution for a time of at least 12 hours.

Preferably, the residence time of the dermis within the enzymatic solution is 24 hours.

During all the residence time of the dermis within the enzymatic solution, the dermis-solution complex is placed within an incubator having controlled atmosphere and temperature.

In particular, the atmosphere of the incubator includes CO₂ in a percentage between 3% and 7%, preferably 5%. The temperature within the incubator is kept between 307 K and 314 K, preferably at 310 K.

In the preferred embodiment, the enzymatic solution consists of a trypsin solution, preferably from a pig, with a dilution between 1.8x and 2.2x in normal saline, for example 0.9% sodium chloride or sterile water.

The preferred trypsin concentration is 2x.

Once the residence time of the dermis within the enzymatic solution is elapsed, the dermis is removed from such solution and dipped in a washing solution.

Such washing solution is a 0.9% sodium chloride sterile solution. The dermis is left in the washing solution for 10 minutes, for the purpose of removing enzymatic solution residues, if any. Following to the washing step of the dermis, the same is dipped in a solution containing culture medium and an adequate mixture of antibiotics and antifungal agents, for the purpose of inactivating enzymatic solution residues which have not been removed by the washing step.

It is to be underlined that the quantity of antibiotic/antifungal solution is at least equal to the quantity of enzymatic solution, so as to ensure an effective inactivation of enzymatic solution residues, if any, still existing on the dermis.

The antibiotic/antifungal solution consists of a 100x mixture of antibiotics and antifungal agents selected, alone or in combination, from the group including 10,000 IU/ml penicillin, 10 mg/ml streptomycin, 25 µ/ml amphotericin B.

The cell culture medium used is RPMI 1640 containing L-glutamine with 25 mM HEPES.

The residence time of the dermis within the antibiotic solution is 10 minutes.

The dermis is successively sealed within bags suitable for the cryofreezing, for example Hermofreeze bags.

It is to be underlined that during the sealing of the dermis within the bags, any additional cryoprotectors is not added to the dermis. In particular, dimethyl sulfoxide is not added.

This occurs in order to not compromising the effectiveness of the successive step, which, in combination with the dipping step in the enzymatic solution above described, allows to obtain an acellular dermis. Such step includes subjecting the dermis to irradiation with ionizing radiations. Such radiations are preferably electromagnetic radiations or particle radiations. Among the electromagnetic radiations, gamma radiations having a frequency higher than 10¹⁹ Hz, preferably between 10¹⁹ and 10²² are preferred.

Among the particle radiations, beta radiations are preferred.

Particle radiations, in particular beta rays, are advantageously produced by an accelerated electron beam preferably produced by a linear or circular accelerator or Rhodotron^{®} or Dynamitron^{®}-type accelerators. The energy of the particle radiations is preferably between 1 and 10 MeV. The power of the beam is advantageously between 1 and 30 kW. The dose (energy per mass unit) is preferably between 50 Gy and 50 kGy.

It is also possible to use a combination of gamma rays and beta rays.

In fact, the irradiation step can also include the minimum presence of other radiations, as a consequence of the type of the radiation-emitting source. Treatments with ionising radiations are carried out for the purpose of reinforcing the action of the enzymatic solution, stabilizing the chemical structures and for the purpose of sterilizing the tissue or organ.

In the realization example described herein, the radiation-emitting source is ¹³⁷Cs which, in addition to the prevailing emission of gamma rays, also emits minimum quantities of beta rays.

The energy per mass unit provided to the tissue by the ionizing electromagnetic radiations is between 90 and 110 Gy, preferably of 100 Gy.

The energy of the ionizing electromagnetic radiations is higher than 100 KeV, preferably between 550 and 750 KeV, more preferably of 660 Kev.

When the irradiation is ended, the bags containing the dermis are cryofrozen, following already known and standardized procedures, at a temperature of about 80 K.

It has been surprisingly noted that the combined action of the enzymatic solution with gamma, beta ionizing radiations or combinations thereof, ensures the removal or the necrotization of any cells responsible for rejection reactions following to a transplantation.

Laboratory tests have pointed out a cell viability equal to 1.8% of the dermis subjected to the combined treatment of enzymatic solution and gamma, beta radiations or combinations thereof (note that in order to define a cellularly viable dermis, the viability value must always be higher than 50%).

At the same time, the combined action of enzymatic solution and gamma, beta radiations or combinations thereof, ensures a perfect biological storage of the extracellular matrix of the dermis.

In other words, the extracellular matrix remains integral in its own protein structure, in its own collagen matrix and in everything except from cells responsible for the rejection reactions. The integrity of the extracellular matrix is both biological (in the sense above described) and mechanical, in the sense that the mechanical properties of the dermis, such as for example elasticity, tensile strength and the like, remain almost unchanged with respect to a dermis which has not undergone any treatment.

Moreover, it has been experimentally noted that the dermis, treated with the combined action of enzymatic solution and gamma, beta radiations or a combination thereof ensures a perfect growth of tenocytes, chondrocytes, stem and mesenchymal cells, thus allowing an optimal cell repopulation and regrowth (with cells of the recipient) when the transplantation has been carried out.

Furthermore, the dermis, thus treated are an optimal support for stem cells or other kinds of cells.

In fact, it is possible to culture stem cells or other kinds of cells (for example hepatocytes, cartilage cells, etc.) on the tissue or the organ treated before the implant within the receiving body.

In this way, a dermis with a better capacity of integration within the receiving organism can be obtained, without giving rise to rejection reactions or minimizing such reactions.

The acellular dermis obtained by the combined action of enzymatic solution and ionizing radiations, in particular gamma and/or beta radiations, finds use in multiple applications.

Examples of such applications include that the acellular dermis obtained by the process above described, and in particular from the combined action of the enzymatic solution and gamma, beta radiations or combnations thereof, is used as a dermal substitute following to burns and/or in the presence of large losses of skin substances and the soft parts (such as for example in the decubitus ulcers), in the chronic ulcers, in the traumatic injuries, in losses of substance of large mucous surfaces, in the reconstruction of the cervical esophagus tract, for neo-vaginae, such as a reinforcement and regeneration of the tendon and ligament injuries, or other tissues of the locomotor apparatus.

The dermis treated with the method of the invention can also be opportunely freeze-dried and then triturated and/or pulverized for being employed as sub-, intradermal or subcutaneous fillers, or in other tissues or organs, as fillers. The acellular dermis can be incised (with laser or mechanical techniques) for carrying out a net-shaped tissue with small meshes, used for reconstructions with platelet gel, growth factors, demineralized bone matrix and the like.

The acellular dermis can also be employed in different forms, among which the tubular one for the muscle, central nervous and peripheral and bone regeneration, and in these uses they can become a tissue regeneration chamber and also containing cells, biological stimulators and signal molecules, grafts and the like.

An additional application includes that the acellular dermis is coated with biocompatible materials, such as for example silicone, polyethylenes, polyurethanes, hydrogels, and charged with different chemical substances and ingredients, in order to carry out a partly artificial dermis consisting of a biological matrix and artificial components.

## Claims

1. Method of decellularization of a dermis withdrawn from a subject including consecutively the following steps;
(i) coating the dermis with an enzymatic solution capable of phagocytizing at least partly fibroblasts, macrophages, mast cells and other dermis-specific cells responsible for immune and rejection reactions in homologous and heterologous transplantations, and
(ii) irradiating the dermis thus treated with ionizing radiations.

2. The method according to claim 1, wherein said enzymatic solution is a trypsin solution.

3. The method according to claim 1 or 2, wherein the step (i) is carried out for at least 12 consecutive hours, preferably for 24 consecutive hours.

4. The method according to any one of the preceding claims, wherein said ionizing radiations are electromagnetic radiations, particle radiations or combinations thereof.

5. The method according to any one of the preceding claims, wherein, said electromagnetic radiations are gamma radiations, having a frequency higher than 10¹⁹ Hz, preferably between 10¹⁹ and 10²² Hz.

6. The method according to any one of the preceding claims, wherein said particle radiations are beta radiations.

7. The method according to claim 6, wherein said particle radiations are irradiated at a dose , constituting an energy per mass unit, between 50 Gy and 50kGy.

8. The method according to any one of the claims from 1 to 7, wherein said step (ii) is performed on dermis sealed in a bag for the cryogenic storage of the biological material.

9. The method according to any one of the claims from 1 to 7, further comprising a step of cryofreezing the dermis after step (ii), the dermis not being subjected to any cryoprotective solutions before the irradiation step.

10. The method according to claim 9, wherein said dermis after the step of cryofreezing is triturated and /or pulverised.

11. The method according to any one of the claims from 1 to 10, wherein said treated dermis is associated or incubated together with stem cells or cells of another type before the implant within the receiving organism, or associated or incubated with autologous, allogenic or synthetic growth factors.

## Patentansprüche

1. Verfahren zur Dezellularisierung einer einer Person entnommenen Dermis, umfassend hintereinander folgende Schritte:
(i) Beschichten der Dermis mit einer enzymatischen Lösung, die in der Lage ist, Fibroblasten, Makrophagen, Mastzellen und sonstige dermisspezifische Zellen, die für Immun- und Abstoßreaktionen bei homologen und heterologen Transplantationen verantwortlich sind, zumindest teilweise zu phagozytieren, und
(ii) Bestrahlen der so behandelten Dermis mit ionisierenden Strahlungen.

2. Verfahren nach Anspruch 1, wobei es sich bei dieser enzymatischen Lösung um eine Trypsinlösung handelt.

3. Verfahren nach Anspruch 1 oder 2, wobei Schritt (i) für mindestens 12 Stunden hintereinander, vorzugsweise 24 Stunden hintereinander, ausgeführt wird.

4. Verfahren nach einem der vorhergehenden Ansprüche, wobei es sich bei den ionisierenden Strahlungen um elektromagnetische Strahlungen, Partikelstrahlungen oder deren Kombinationen handelt.

5. Verfahren nach einem der vorhergehenden Ansprüche, wobei es sich bei diesen elektromagnetischen Strahlungen um Gammastrahlungen handelt, die eine Frequenz über 10¹⁹ Hz aufweisen, vorzugsweise zwischen 10¹⁹ und 10²² Hz.

6. Verfahren nach einem der vorhergehenden Ansprüche, wobei es sich bei den Partikelstrahlungen um Beta-Strahlungen handelt.

7. Verfahren nach Anspruch 6, wobei die Partikelstrahlungen bei einer Dosierung abgegeben werden, darstellend eine Energie pro Masseneinheit zwischen 50 Gy und 50 kGy.

8. Verfahren nach einem der Ansprüche von 1 bis 7, wobei Schritt (ii) auf einer in einem Beutel verschlossenen Dermis für die kryogene Lagerung des biologischen Materials durchgeführt wird.

9. Verfahren nach einem der vorhergehenden Ansprüche von 1 bis 7, zudem umfassend einen Schritt zum Kryokonservieren der Dermis nach Schritt (ii), wobei die Dermis vor dem Bestrahlungsschritt keinen Kryoschutzlösungen unterzogen wird.

10. Verfahren nach Anspruch 9, wobei die Dermis nach dem Schritt des Kryokonservierens zermahlen und/oder pulverisiert wird.

11. Verfahren nach einem der Ansprüche 1 bis 10, wobei die behandelte Dermis vor der Implantation in den Empfängerorganismus mit Stammzellen oder Zellen eines anderen Typs verbunden oder inkubiert oder mit autologen, allogenen oder synthetischen Wachstumsfaktoren verbunden oder inkubiert wird.

## Revendications

1. Procédé de décellularisation d'un derme tiré d'un sujet incluant les étapes consécutives suivantes :
(i) revêtir le derme d'une solution enzymatique en mesure de phagocyter au moins en partie les fibroblastes, macrophages, mastocytes et autres cellules spécifiques du derme responsables des réactions immunitaires ou de rejet de greffe dans une allogreffe et xénogreffe, et
(ii) irradier le derme ainsi traité par rayonnements ionisants.

2. Procédé selon la revendication 1, dans lequel ladite solution enzymatique est une solution de trypsine.

3. Procédé selon les revendications 1 ou 2, dans lequel l'étape (i) s'effectue pendant au moins 12 heures consécutives, de préférence pendant 24 heures consécutives.

4. Procédé selon l'une quelconque des revendications précédentes, dans lequel lesdits rayonnements ionisants sont des rayonnements électromagnétiques, des rayonnements corpusculaires ou leurs combinaisons.

5. Procédé selon l'une quelconque des revendications précédentes, dans lequel lesdits rayonnements électromagnétiques sont des rayonnements gamma ayant une fréquence supérieure à 10¹⁹ Hz, de préférence entre 10¹⁹ et 10²² Hz.

6. Procédé selon l'une quelconque des revendications précédentes, dans lequel lesdits rayonnements corpusculaires sont des rayonnements bêta.

7. Procédé selon la revendication 6, dans lequel lesdits rayonnements corpusculaires sont irradiés avec une dose constituant une énergie par unité de masse comprise entre 50 Gy et 50 kGy.

8. Procédé selon l'une quelconque des revendications de 1 à 7, dans lequel ladite étape (ii) s'effectue sur un derme placé dans un sac hermétique destiné au stockage cryogénique du matériel biologique.

9. Procédé selon l'une quelconque des revendications de 1 à 7, comprenant de plus une étape de cryo-congélation du derme après l'étape (ii), le derme n'étant soumis à aucune solution cryoprotectrice avant l'étape d'irradiation.

10. Procédé selon la revendication 9, dans lequel ledit derme après l'étape de cryo-congélation est trituré et / ou pulvérisé.

11. Procédé selon l'une quelconque des revendications précédente de 1 à 10, dans lequel ledit derme traité est associé ou étuvé avec des cellules souches ou des cellules d'un autre type avant l'implant à l'intérieur de l'organisme receveur ou associé ou étuvé avec des facteurs de croissance autologues, allogéniques ou synthétiques.
